# EUROPEAN PATENT SPECIFICATION

(11) **EP 1 696 023 B1**
(45) Date of publication and mention of the grant of the patent: **16.04.2008**
(21) Application number: 06003925.2
(22) Date of filing: 27.02.2006
(51) Int. Cl.: C11D 1/29, C11D 1/14, C11D 1/83, C11D 1/72, C11D 3/02, C11D 3/20, C11D 11/00

(54) **Surfactant composition**
Tensidzusammensetzung
Composition tensioactive

(30) Priority: 28.02.2005 JP 2005053737
(43) Date of publication of application: 30.08.2006
(73) Proprietor: KAO CORPORATION, Chuo-ku Tokyo 103-8210 (JP)
(72) Inventor: Inoue, Masaki, Wakayama-shi Wakayama 640-8580 (JP); Doi, Yasuhiro, Wakayama-shi Wakayama 640-8580 (JP)
(74) Representative: HOFFMANN EITLE

(56) References cited:
- EP-A- 1 013 754
- EP-A- 1 092 761
- EP-A- 1 226 814
- WO-A-95/14073
- US-A- 4 412 945

## Description

### [Field of the Invention]

The present invention relates to a surfactant composition useful for the preparation of a skin detergent or hair detergent such as shampoo or body shampoo which composition exhibits, in spite of containing a polyoxyethylene alkyl ether sulfate or alkyl sulfate in a high concentration, good fluidity over a wide temperature range, causes neither thickening nor gelation during dilution, and can maintain the intrinsic performance of the surfactant.

### [Background of the Invention]

Compositions containing an ordinarily employed polyoxyethylene alkyl ether sulfate or alkyl sulfate in a high concentration show poor fluidity and therefore cannot be handled readily. To provide easy handling, they are used in a concentration of not more than 30 wt.% or in a very low viscosity range. Various attempts have been made to add a third component (viscosity decreasing agent) which does not affect the performance, thereby increasing the concentration or decreasing the viscosity whatever the objective of use of a polyoxyethylene alkyl ether sulfate or alkyl sulfate is. For example, many methods have been proposed such as a method of adding a sulfonate salt of an unsaturated fatty acid polyalkylene alkyl ester and a polyalkylene alkyl ester salt of α-sulfonic acid (JP-A-10-17898), a method of adding an alkyl glucoside and an alkali metal salt (WO 91/04313) and a method of adding polyoxyethylene alkyl ether having an average molecular weight of 4,000 to 10,000 and an alkyl group of 8 to 16 carbon atoms (US4412945). These methods are however not suited for some objectives of use owing to their adverse effect on the performances such as foamability, and moreover, formulation of a detergent must be carried out under heating to improve the fluidity at low temperatures. These methods are therefore not satisfactory.

The method disclosed in JP-A-10-17898 or WO 91/04313 is described to be effective for improving the fluidity in a temperature range from about room temperature (20°C) to 70°C, but these documents do not include a description as to the fluidity improving effect at a low temperature (5°C).

### [Summary of the Invention]

As described above, compositions containing a polyoxyethylene alkyl ether sulfate or alkyl sulfate in a high concentration are thick, have poor fluidity and therefore cannot be handled easily. In addition to these problems, such compositions gelate when they are diluted upon use.

An object of the present invention is therefore to provide a surfactant composition useful for the preparation of a skin detergent or hair detergent such as shampoo or body shampoo which composition, in spite of containing a polyoxyethylene alkyl ether sulfate or alkyl sulfate in a high concentration, exhibits good fluidity over a wide temperature range, causes neither thickening nor gelation during dilution and can maintain the intrinsic performance of the surfactant.

The present inventors have found that by using a specific glyceryl ether or diglyceryl ether and a water-soluble salt in combination with a polyoxyethylene alkyl ether sulfate or alkyl sulfate, it is possible to obtain a surfactant composition useful for the preparation of a skin detergent or hair detergent such as shampoo or body shampoo which composition exhibits good fluidity over a wide temperature range, causes neither thickening nor gelation during dilution and can maintain the intrinsic performance of the surfactant.

According to the present invention, there is thus provided a surfactant composition, which contains the following components (A), (B) and (C):
(A) a polyoxyethylene alkyl ether sulfate or alkyl sulfate (which may hereinafter be called "Component (A)") represented by the following formula (1):

   R¹O (CH₂CH₂O)ₙSO₃M (1)

   (wherein, R¹ represents a saturated or unsaturated hydrocarbon group having 8 to 18 carbon atoms, "n", which is an average number of moles added, is from 0 to 5, and M represents an alkali metal atom, alkanolamine or ammonium;
(B) one or more glyceryl ethers or diglyceryl ethers (which may hereinafter be called "Component (B)") having an alkyl or alkenyl group having 4 to 24 carbon atoms, and
(C) a water-soluble salt (which may hereinafter be called "Component (C)"), wherein the content of Component (A) is not less than 40 wt.%.

According to the present invention, it is possible to provide a surfactant composition useful for the preparation of a skin detergent or hair detergent such as shampoo or body shampoo which composition, in spite of containing a polyoxyethylene alkyl ether sulfate or alkyl sulfate in a high concentration, exhibits good fluidity over a wide temperature range, causes neither thickening nor gelation during dilution, and can maintain the intrinsic performance of the surfactant such as foamability and feeling upon use.

### [Detailed Description of the Invention]

The present invention will next be described more specifically.
In the polyoxyethylene alkyl ether sulfate or alkyl ether sulfate represented by the formula (1), which is used as Component (A) in the invention, R¹ represents a saturated or unsaturated hydrocarbon group having 8 to 18 carbon atoms, preferably 10 to 16 carbon atoms, more preferably from 12 to 14 carbon atoms and is preferably a saturated group. The hydrocarbon group of R¹ may be linear or branched, but is preferably linear. The average number of moles added ranges from 0 to 5, preferably from 1 to 3, more preferably 1 to 2. M represents an alkali metal atom, alkanolamine or ammonium. Examples of the alkali metal atom include sodium and potassium, with sodium being preferred. As the alkanolamine, triethanolamine is preferred.

Component (A) is incorporated in the surfactant composition preferably in an amount of from 40 to 70 wt.%, more preferably from 45 to 65 wt.% from the standpoint of fluidity.

The glyceryl ether or diglyceryl ether (preferably glyceryl ether) as Component (B) has a linear or branched alkyl or alkenyl group having 4 to 24 carbon atoms. Those having an alkyl group of 4 to 12 carbon atoms such as n-butyl, isobutyl, tert-butyl, n-pentyl, isopentyl, n-hexyl, isohexyl, n-heptyl, n-octyl, 2-ethylhexyl, n-nonyl, n-decyl, isodecyl or n-lauryl group are preferred, with those having one or two alkyl groups, especially one alkyl group of 4 to 11 carbon atoms, particularly 6 to 10 carbon atoms being still more preferred. Of them, those having one or two alkyl groups, particularly one alkyl group of 8 carbon atoms (especially 2-ethylhexyl group) are preferred.

As Component (B), one or more of the above-described compounds may be used. Component (A) and Component (B) may be added to the surfactant composition of the present invention at an (A)/(B) weight ratio of from 99 /l to 75/25, preferably from 98/2 to 85/15, more preferably from 93/3 to 90/10 from the standpoint of the fluidity improving effects when Component (A) is added at a concentration of not less than 40 wt.%. Within the above-described range, the surfactant composition can exhibit sufficient fluidity-improved effects.

Component (B) is added to the surfactant composition preferably in an amount of from 0.5 to 15 wt.%, more preferably from 1 to 10 wt.%, still more preferably from 2 to 8 wt.% in order to improve the fluidity particularly at low temperatures.

As the water-soluble salt as Component (C), one or more water-soluble salts selected from water-soluble inorganic salts and water-soluble organic salts other than surfactants are preferred. Examples thereof include salts of organic acids such as citric acid, malic acid, succinic acid and lactic acid and salts of inorganic acids such as hydrochloric acid, sulfuric acid, nitric acid, carbonic acid and phosphoric acid. Examples of the cation forming these salts include alkali metals such as sodium and potassium, alkaline earth metals such as calcium and magnesium, as well as ammonium and aluminum. Specific preferred examples of such salts include alkali metal salts of an inorganic acid such as sodium chloride and sodium sulfate; ammonium salts of an inorganic acid such as ammonium chloride, ammonium sulfate and ammonium nitrate; alkali metal salts of an organic acid (especially preferably an aliphatic organic acid) such as trisodium citrate; and ammonium salts of an organic acid (especially preferably an aliphatic organic acid). Alkali metal salts of an inorganic acid and ammonium salts of an inorganic acid are preferred. One or more of these salts may be used. Component (A) and Component (C) are incorporated in the surfactant composition of the present invention at an (A) / (C) weight ratio of from 99/1 to 85/15, more preferably from 99/1 to 90/10, still more preferably from 98/2 to 95/5 in order to improve the fluidity of the composition containing Component (A) at a concentration of not less than 40 wt.%. Within the above-described range, the composition can exhibit sufficient fluidity-improved effects.

Component (C) is incorporated preferably in an amount of from 0.1 to 10 wt.%, more preferably from 0.2 to 8 wt.%, even more preferably from 0.5 to 5 wt.%, still more preferably from 1 to 4 wt.% in the surfactant composition from the standpoint of the fluidity of the composition.

The surfactant composition of the present invention preferably has a viscosity at 5°C of not more than 90000 mPa·s, more preferably from 1000 to 70000 mPa·s, still more preferably from 5000 to 50000 mPa·s from the standpoint of improved fluidity and excellent handling properties of the composition.
The surfactant composition of the present invention can be prepared by mixing Components (A), (B) and (C) under stirring at from 15 to 60°C. Although no particular limitation is imposed on the form of the composition, a liquid form, paste form or cream form is preferred. Use of a solvent for the preparation of the composition is preferred and, as the solvent, water is preferred.

The surfactant composition of the present invention can be used as a detergent composition after being diluted with water. For example, it can be incorporated in a detergent composition for skin detergents such as face wash and body shampoo or hair detergents such as shampoo. In a detergent composition having the surfactant composition incorporated therein, optional components can be incorporated depending on the objective of use. Examples of the optional components include anionic surfactants other than Component (A), nonionic surfactants, amphoteric surfactants, cationic surfactants and conditioning agents, which are conventionally added to such detergents. Components (A), (B) and (C) of the present invention may also be added if necessary.

Examples of the anionic surfactant other than Component (A) include fatty acid salts, phosphate ester salts, sulfosuccinic acid surfactants, polyoxyalkylene alkyl amide ether sulfates, monoglyceride sulfates, olefin sulfonates, alkanesulfonates, acylated isethionates, acylated amino acid salts, polyoxyalkylene alkyl ether phosphates and polyoxyalkylene alkyl ether acetates.

Examples of the nonionic surfactant include alkyl polyglycosides, sucrose fatty acid esters, polyglycerin fatty acid esters, polyoxyalkylene alkyl ethers, fatty acid alkanolamides, alkylamine oxides, and fatty acid polyol esters.

Examples of the amphoteric surfactant include amidobetaine surfactants, amide amino acid surfactants, carbobetaine surfactants, sulfobetaine surfactants, amidosulfobetaine surfactants, imidazolinium betaine surfactants and phosphobetaine surfactants.

Example of the cationic surfactant include quaternary ammonium salts represented by the following formula (2):

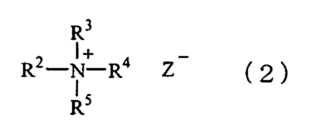

wherein, at least one of R², R³, R⁴ and R⁵ represents an alkyl or alkenyl group which may be substituted by an alkoxy group, alkenyloxy group, alkanoylamino group or alkenoylamino group having 8 to 28 carbon atoms in total, the remaining group(s) each represents a benzyl group, an alkyl or hydroxyl group having 1 to 5 carbon atoms or a polyoxyethylene group whose number of moles added is not more than 10 in total, and Z⁻ represents a halogen ion or an organic anion.

Examples of the conditioning component include oil components, for example, higher alcohols such as lauryl alcohol, myristyl alcohol, cetyl alcohol, and stearyl alcohol, silicones, silicone derivatives, lanolin, squalene, hydrocarbons, protein derivatives, and polyethylene glycol fatty acid esters, and cationic polymers such as cationized cellulose, cationized guar gum and "Merquat 550" (product of Merck & Co.), and cationic group-containing copolymers such as "Sofcare KG-301W" (product of Kao Corp.).

The detergent composition prepared according to the present invention may contain, within an extent not impairing the advantage of the present invention, other conventionally used components, for example, water-soluble polymers, e.g., polysaccharides such as methyl cellulose, hydroxyethyl cellulose, carboxyvinyl polymers or xanthan gum; viscosity regulators such as polyoxyalkylene sorbitan esters, polyoxyethylene glycol distearates or ethanol; chelating agents such as ethylenediamine tetraacetic acid (EDTA) or phosphonate salts; antiseptics such as methylparaben or butylparaben; effective components such as vitamins or precursors thereof; extracts derived from animals or plants such as lecithin or gelatin, or derivatives of the extracts; polymer fine particles such as nylon or polyethylene; anti-inflammatory agents such as dipotassium glycyrrhizinate; bactericides or antidandruffs such as triclosan, triclocarban, octopirox or zinc pyrithione; antioxidant such as dibutylhydroxytoluene; pearling agents; ultraviolet absorbers; pH regulators; colorants; perfumes; or water.

The detergent composition prepared according to the present invention can be prepared in a manner known *per se* in the art. No particular limitation is imposed on the form of the detergent composition and it can be provided in a desired form such as liquid, paste, cream, solid or powder. Preferred forms include liquid, paste and cream, with liquid being especially preferred. When the composition is provided in the liquid form, water is preferably used as a liquid medium.

### [Examples]

The present invention will hereinafter be described more specifically by way of examples.

### Examples 1 to 26 and Comparative Examples 1 to 12.

Surfactant compositions as shown in Tables 1 to 3 were prepared in a manner *per se* in the art. By the below-described method, the viscosity and fluidity of the surfactant composition at 30°C and at 5°C, and foamability of an aqueous solution containing 15 wt.% of Component (A) were evaluated.
When the surfactant compositions of Examples 1 to 26 were diluted to adjust the concentration of Component (A) to 15 wt.%, dilution was carried out easily (within 5 minutes) at room temperature scarcely causing gelation. When the surfactant compositions of Comparative Examples 1 to 7 and 10 were diluted to adjust the concentration of Component (A) to 15 wt.%, on the other hand, gelation occurred and it took a long time (about 1 hour) for dilution at room temperature. When the surfactant compositions of Comparative Examples 8, 9 and 11 were diluted to adjust the concentration of Component (A) to 15 wt.%, a little gelation occurred and it took a relatively long time (15 to 30 minute) for dilution at room temperature.

### (1) Measurement of viscosity

Viscosity was measured under the following conditions. Viscometer: B-type viscometer (product of Tokyo Keiki) Rotor No./rotation speed: No. 4/6 rpm, measured for 1 minute.

### Temperature:

30°C: Viscosity was measured after a glass bottle containing a sample therein was soaked for 1 hour in a thermostat bath at 30 ± 1°C.
5°C: Viscosity was measured immediately after the glass bottle soaked in a thermostat at 5 ± 1°C for 24 hours was taken out.

### (2) Fluidity

Fluidity was visually evaluated while inclining a glass bottle containing a surfactant composition therein by 90°.

### Temperature:

30°C: Fluidity was measured after a glass bottle containing a sample therein was soaked for 1 hour in a thermostat bath at 30 ± 1°C.
5°C: Fluidity was measured immediately after the glass bottle soaked in a thermostat at 5 ± 1°C for 24 hours was taken out.

### (Fluidity)

A: Good fluidity
B: Slight fluidity
C: No fluidity

### (3) Test on foamability

A panel of 10 experts dropped 1 mL of a surfactant composition which had been diluted to adjust the concentration of Component A to 15 wt.% onto the palm and washed their hands and arms with the composition. The foamability of the surfactant composition at that time was compared with that of a control composition [15% aqueous solution of sodium polyoxyethylene alkyl ether sulfate (n=2 moles)] and evaluated based on the following standards.

### (Foamability)

4: A foam volume is much greater than that of the control composition.
3: A foam volume is greater than that of the control composition.
2: A foam volume is equal to that of the control composition.
1: A foam volume is smaller than that of the control composition.
An average score of 10 experts was calculated and the compositions having a score of not less than 3.6, a score of from 2.6 to 3.5, a score of from 1.6 to 2.5, and a score of not more than 1.5 were ranked as A, B, C, and D, respectively.

**[Table 1]**

| Component (wt%) | | Examples | | | | | | | | | | | | | |
|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 |
| (A) | Ammonium polyoxyethylene alkyl ether sulfate (n = 1 mole) | 64 | 64 | 64 | | | | 64 | 60 | 55 | 60 | 60 | 45 | 45 | 45 |
| | Ammonium polyoxyethylene alkyl ether sulfate (n = 2 moles) | | | | 64 | | | | | | | | | | |
| | Sodium polyoxyethylene alkyl ether sulfate (n = 2 moles) | | | | | 60 | | | | | | | | | |
| | Triethanolamine polyoxyethylene alkyl ether sulfate (n= 3 moles) | | | | | | 64 | | | | | | | | |
| | Ammonium alkyl sulfate | | | | | | | | | | | | | | |
| | Propylene glycol | | | | | | | | | | | | | | |
| | Polyglycerin ethoxylate (Mw 6000) | | | | | | | | | | | | | | |
| | Ethanol | | | | | | | | | | | | | | |
| | Polyethylene glycol (Mw 600) | | | | | | | | | | | | | | |
| | Polyethylene glycol (Mw 600) disulfate | | | | | | | | | | | | | | |
| | Glycerin monocaprylate | | | | | | | | | | | | | | |
| | Decyl glucoside | | | | | | | | | | | | | | |
| (B) | 2-Ethylhexyl glyceryl ether | 2.0 | 3.4 | 7.1 | | 3.2 | 3.4 | 3.4 | 3.2 | 2.9 | 3.2 | 3.2 | 2.4 | 2.4 | 2.4 |
| | Isodecyl glyceryl ether | | | | | | | | | | | | | | |
| | Isostearyl glyceryl ether | | | | | | | | | | | | | | |
| | Pentyl glyceryl ether | | | | 2.0 | | | | | | | | | | |
| | 2-Ethylhexyl diglyceryl ether | | | | | | | | | | | | | | |
| (C) | Sodium chloride | 1.3 | 1.3 | 1.3 | 1.3 | 1.2 | 0.5 | | 2.5 | | | | 1.9 | 1.4 | |
| | Sodium sulfate | | | | | | | | | | | | | | |
| | Sodium carbonate | | | | | | | 2 | | | | | | | |
| | Trisodium citrate | | | | | | | | | | | | | | |
| | Sodium lactate | | | | | | | | | | | | | | |
| | Ammonium chloride | | | | | | | | | 1.7 | | 1.9 | | | 1.4 |
| | Ammonium sulfate | | | | | | | | | | 1.9 | | | | |
| | Ammonium nitrate | | | | | | | | | | | | | | |
| Purified water | | Balance | Balance | Balance | Balance | Balance | Balance | Balance | Balance | Balance | Balance | Balance | Balance | Balance | Balance |
| (A)/(B) | | 97/3 | 95/5 | 90/10 | 97/3 | 95/5 | 95/5 | 95/5 | 95/5 | 95/5 | 95/5 | 95/5 | 95/5 | 95/5 | 95/5 |
| (A)/(C) | | 98/2 | 98/2 | 98/2 | 98/2 | 98/2 | 98/2 | 97/3 | 96/4 | 97/3 | 97/3 | 97/3 | 96/4 | 97/3 | 97/3 |
| Viscosity (mPa·s, 30°C) | | 12500 | 16000 | 22000 | 13500 | 22000 | 22500 | 14000 | 32500 | 7500 | 13500 | 10500 | 6000 | 5200 | 5500 |
| Viscosity (mPa·s, 5°C) | | 17000 | 25000 | 30000 | 17000 | 49000 | 37000 | 18000 | 33000 | 12000 | 17000 | 17000 | 9000 | 9000 | 7000 |
| Fluidity at 30°C | | A | A | A | A | A | B | A | A | A | A | A | A | A | A |
| Fluidity at 5°C | | B | A | A | A | A | B | A | A | A | A | A | A | A | A |
| Foamability (15% aqueous solution) | | B | A | A | B | A | B | A | A | A | A | A | A | A | A |

The alkyl group of the polyoxyethylene alkyl ether sulfate or alkyl sulfate as Component (A) is linear and has 12 to 14 carbon atoms; n means the average number of moles of ethylene oxide added.

**[Table 2]**

| Component (wt.%) | | Examples | | | | | | | | | | | |
|---|---|---|---|---|---|---|---|---|---|---|---|---|---|
| | | 15 | 16 | 17 | 18 | 19 | 20 | 21 | 22 | 23 | 24 | 25 | 26 |
| (A) | Ammonium polyoxyethylene alkyl ether sulfate (n = 1 mole) | 64 | 64 | 64 | 64 | 64 | 60 | | 60 | 60 | | | 45 |
| | Ammonium polyoxyethylene alkyl ether sulfate (n = 2 moles) | | | | | | | 64 | | | 64 | | |
| | Sodium polyoxyethylene alkyl ether sulfate (n = 2 moles) | | | | | | | | | | | | |
| | Triethanolamine polyoxyethylene alkyl ether sulfate (n = 3 moles) | | | | | | | | | | | | |
| | Ammonium alkyl sulfate | | | | | | | | | | | 45 | |
| | Propylene glycol | | | | | | | | | | | | |
| | Polyglycerin ethoxylate (Mw 6000) | | | | | | | | | | | | |
| | Ethanol | | | | | | | | | | | | |
| | Polyethylene glycol (Mw 600) | | | | | | | | | | | | |
| | Polyethylene glycol (Mw 600) disulfate | | | | | | | | | | | | |
| | Glycerin monocaprylate | | | | | | | | | | | | |
| | Decyl glucoside | | | | | | | | | | | | |
| (B) | 2-Ethylhexyl glyceryl ether | | | | | | | | 3.2 | 3.2 | 3.2 | 5.0 | 2.2 |
| | Isodecyl glyceryl ether | 3.4 | | | | | | | | | | | |
| | Isostearyl glyceryl ether | | 3.4 | | 7.1 | | | | | | | | |
| | Pentyl glyceryl ether | | | 3.4 | | 7.1 | | | | | | | |
| | 2-Ethylhexyl diglyceryl ether | | | | | | 3.2 | 3.4 | | | | | |
| (C) | Sodium chloride | 1.3 | 1.3 | 1.3 | 1.3 | 1.3 | | 1.3 | | | 1.2 | | |
| | Sodium sulfate | | | | | | | | | | | | |
| | Sodium carbonate | | | | | | | | | | | | |
| | Trisodium citrate | | | | | | | | 1.9 | | | | |
| | Sodium lactate | | | | | | | | | | | | 1.4 |
| | Ammonium chloride | | | | | | 6.7 | | | | | 4 | |
| | Ammonium sulfate | | | | | | | | | | | | |
| | Ammonium nitrate | | | | | | | | | 1.9 | | | |
| Purified water | | Balance | Balance | Balance | Balance | Balance | Balance | Balance | Balance | Balance | Balance | Balance | Balance |
| (A)/(B) | | 95/5 | 95/5 | 95/5 | 90/10 | 90/10 | 95/5 | 95/5 | 95/5 | 95/5 | 95/5 | 90/10 | 95/5 |
| (A)/(C) | | 98/2 | 98/2 | 98/2 | 98/2 | 98/2 | 90/10 | 98/2 | 97/3 | 97/3 | 98/2 | 92/8 | 97/3 |
| Viscosity (mPa·s, 30°C) | | 14000 | 14000 | 14000 | 15500 | 15500 | 8000 | 16500 | 11000 | 12300 | 21500 | 20500 | 12500 |
| Viscosity (mPa·s, 5°C) | | 30000 | 19000 | 18000 | 23000 | 20500 | 8000 | 20000 | 17000 | 22500 | 29000 | 39000 | 20000 |
| Fluidity at 30°C | | A | A | A | A | A | A | A | A | A | A | B | A |
| Fluidity at 5°C | | B | B | B | A | A | A | B | A | A | B | B | A |
| Foamability (15% aqueous solution) | | A | B | B | A | B | B | A | A | A | B | B | B |

The alkyl group of the polyoxyethylene alkyl ether sulfate or alkyl sulfate as Component (A) is linear and has 12 to 14 carbon atoms; n means the average number of moles of ethylene oxide added.

**[Table 3]**

| Component (wt.%) | | Comparative Examples | | | | | | | | | | | |
|---|---|---|---|---|---|---|---|---|---|---|---|---|---|
| | | 1 | 2 | 3 | 4 | 5 | 6 | 7 | 8 | 9 | 10 | 11 | 12 |
| (A) | Ammonium polyoxyethylene alkyl ether sulfate (n = 1 mole) | 64 | 60 | 64 | 64 | 64 | 64 | 64 | 64 | 64 | | | 64 |
| | Ammonium polyoxyethylene alkyl ether sulfate (n = 2 moles) | | | | | | | | | | 60 | 60 | |
| | Sodium polyoxyethylene alkyl ether sulfate (n = 2 moles) | | | | | | | | | | | | |
| | Triethanolamine polyoxyethylene alkyl ether sulfate (n = 3 moles) | | | | | | | | | | | | |
| | Ammonium alkyl sulfate | | | | | | | | | | | | |
| | Propylene glycol | | | 3.4 | . | | | | | | | | |
| | Polyglycerin ethoxylate (Mw 6000) | | | | 3.4 | | | | | | | | |
| | Ethanol | | | | | 3.4 | | | | | | | |
| | Polyethylene glycol (Mw 600) | | | | | | 3.4 | | | | | | |
| | Polyethylene glycol (Mw 600) disulfate | | | | | | | | | | | | 3.4 |
| | Glycerin monocaprylate | | | | | | | 3.4 | | | | | |
| | Decyl glucoside | | | | | | | | 3.4 | | | | |
| (B) | 2-Ethylhexyl glyceryl ether | | | | | | | | | | | | |
| | Isodecyl glyceryl ether | | | | | | | | | | | | |
| | Isostearyl glyceryl ether | | | | | | | | | | | | |
| | Pentyl glyceryl ether | | | | | | | | | | | | |
| | 2-Ethylhexyl diglyceryl ether | | | | | | | | | | | | |
| (C) | Sodium chloride | | | | | | | | 1.3 | 1.3 | | | |
| | Sodium sulfate | | | | | | | | | | | 1.2 | |
| | Sodium carbonate | | | | | | | | | | | | |
| | Trisodium citrate | | | | | | | | | | | | |
| | Sodium lactate | | | | | | | | | | | | |
| | Ammonium chloride | | | | | | | | | | | | |
| | Ammonium sulfate | | | | | | | | | | | | |
| | Ammonium nitrate | | | | | | | | | | | | |
| Purified water | | Balance | Balance | Balance | Balance | Balance | Balance | Balance | Balance | Balance | Balance | Balance | Balance |
| (A)/(B) | | 100/0 | 100/0 | 100/0 | 100/0 | 100/0 | 100/0 | 100/0 | 100/0 | 100/0 | 100/0 | 100/0 | 100/0 |
| (A)/(C) | | 100/0 | 100/0 | 100/0 | 10010 | 100/0 | 100/0 | 100/0 | 98/2 | 98/2 | 100/0 | 98/2 | 100/0 |
| Viscosity (mPa·s, 30°C) | | 24000 | 100000 | 18000 | 16000 | 16500 | 16000 | 14500 | 14000 | 13500 | 100000 | 24000 | 18000 |
| Viscosity (mPa·s, 5°C) | | 100000 ≤ | 100000 ≤ | 100000 ≤ | 100000 ≤ | 100000 ≤ | 100000 ≤ | 100000 ≤ | 100000 ≤ | 100000 ≤ | 100000 ≤ | 100000 ≤ | 100000 ≤ |
| Fluidity at 30°C | | B | C | A | A | B | B | B | A | A | C | B | B |
| Fluidity at 5°C | | C | C | C | C | C | C | C | C | C | C | C | C |
| Foamability (15% aqueous solution) | | C | C | D | D | D | D | C | C | C | C | C | C |

The alkyl group of the polyoxyethylene alkyl ether sulfate or alkyl sulfate as Component (A) is linear and has 12 to 14 carbon atoms; n means the average number of moles of ethylene oxide added.

### Example 27

A body shampoo having the below-described composition was prepared.

| | | |
|---|---|---|
| Surfactant composition of Example 5 | | 5.0 |
| Sodium laurate | | 6.0 |
| Sodium myristate | | 3.0 |
| Sodium palmitate | | 1.0 |
| 2-Ethylhexyl glyceryl ether | | 1.0 |
| Glycerin | | 3.0 |
| Methylparaben | | q.s. |
| Perfume | | q.s. |
| Purified water | | Balance |
| | Total | 100.0 |

The resulting body shampoo was prepared readily without causing a viscosity increase during dilution of the surfactant composition of Example 5, exhibited a good foamability and was excellent in feeling upon use.

### Example 28

A shampoo having the below-described composition was prepared.

| | | |
|---|---|---|
| Surfactant composition of Example 13 | | 15.0 |
| Amidopropylbetaine * | | 2.0 |
| Myristyl alcohol | | 1.0 |
| Cationic group-containing copolymer ** | | 0.2 |
| Ethanol | | 3.0 |
| Perfume | | q.s. |
| Purified water | | Balance |
| | Total | 100.0 |

| | | |
|---|---|---|
| *: "Amphitol 20AB" (product of Kao Corp.) **: "Sofcare KG-301W" (product of Kao Corp.) | | |

The resulting shampoo was prepared readily without causing a viscosity increase during dilution of the surfactant composition of Example 13, exhibited a good foamability and was excellent in feeling upon use.

### Example 29

A conditioning shampoo having the below-described composition was prepared.

| | | |
|---|---|---|
| Surfactant Composition of Example 13 | | 12.0 |
| Coconut oil fatty acid N-methylethanolamide * | | 0.8 |
| Myristyl alcohol | | 1.0 |
| Cationic group-containing copolymer ** | | 0.2 |
| Cationized cellulose *** | | 0.4 |
| Cationized guar gum **** | | 0.2 |
| Silicone ***** | | 1.0 |
| Perfume | | q.s. |
| Purified water | | Balance |
| | Total | 100.0 |

| | | |
|---|---|---|
| *: "Aminon C-11S" (product of Kao Corp.) **: "Sofcare KG-301W" (product of Kao Corp.) ***: "Poiz C-150L" (product of Kao Corp.) ****: "JAGUAR C-13S" (product of Rhodia) *****: "BY 22-060" (product of Dow Corning Toray) | | |

The resulting conditioning shampoo was prepared readily without causing a viscosity increase during dilution of the surfactant composition of Example 13, exhibited a good foamability and was excellent in feeling upon use.

## Claims

1. A surfactant composition comprising the following components (A), (B) and (C):
(A) a polyoxyethylene alkyl ether sulfate or alkyl sulfate represented by the following formula (1):
R¹O(CH₂CH₂O)ₙSO₃M (1)
wherein, R¹ represents a saturated or unsaturated hydrocarbon group having 8 to 18 carbon atoms, "n", which is an average number of moles added, is a number of from 0 to 5, and M represents an alkali metal atom, an alkanolamine or ammonium;
(B) a glyceryl ether or diglyceryl ether having an alkyl or alkenyl group having 4 to 24 carbon atoms, and
(C) a water-soluble salt, wherein the content of Component (A) is not less than 40 wt.%.

2. The surfactant composition according to Claim 1, wherein Component (A) and Component (B) are added at an (A)/(B) weight ratio in a range of from 99/1 to 75/25.

3. The surfactant composition according to Claim 1, wherein Component (A) and Component (C) are added at a (A)/(C) weight ratio in a range of from 99/1 to 85/15.

4. The surfactant composition according to any one of Claims 1 to 3, having a viscosity at 5°C of not more than 90000 mPa·s.

5. The surfactant composition according to any one of Claims 1 to 4, wherein the water-soluble salt is at least one selected from water-soluble inorganic salts and water-soluble organic salts other than surfactants.

6. The surfactant composition according to any one of Claims 1 to 4, wherein the water-soluble salt is at least one selected from alkali metal salts of an inorganic acid and ammonium salts of an inorganic acid.

7. A process for preparation of a detergent composition, which comprises incorporating a surfactant and/or water in the surfactant composition as claimed in any one of Claims 1 to 6.

8. Use of the surfactant composition as claimed in any one of Claims 1 to 6 for the preparation of a detergent composition.

9. The use according to Claim 8, wherein a surfactant and/or water is additionally incorporated.

## Patentansprüche

1. Tensidzusammensetzung umfassend die folgenden Komponenten (A), (B) und (C):
(A) ein Polyoxyethylenalkylethersulfat oder Alkylsulfat, das durch die folgende Formel (1) dargestellt wird:
R¹O(CH₂CH₂O)ₙSO₃M (1)
wobei R¹ eine gesättigte oder ungesättigte Kohlenwasserstoffgruppe mit 8 bis 18 Kohlenstoffatomen darstellt, n, das eine mittlere Zahl der zugesetzten Mole ist, eine ganze Zahl von 0 bis 5 ist und M ein Alkalimetallatom, ein Alkanolamin oder Ammonium darstellt;
(B) ein Glycerylether oder Diglycerylether mit einer Alkyl- oder Alkenylgruppe mit 4 bis 24 Kohlenstoffatomen und
(C) ein wasserlösliches Salz,
wobei der Gehalt an Komponente (A) nicht weniger als 40 Gew.% beträgt.

2. Tensidzusammensetzung gemäß Anspruch 1, wobei Komponente (A) und Komponente (B) in einem Gewichtsverhältnis (A)/(B) in einem Bereich von 99/1 bis 75/25 zugesetzt werden.

3. Tensidzusammensetzung gemäß Anspruch 1, wobei Komponente (A) und Komponente (C) in einem Gewichtsverhältnis (A)/(C) in einem Bereich von 99/1 bis 85/15 zugesetzt werden.

4. Tensidzusammensetzung gemäß einem der Ansprüche 1 bis 3 mit einer Viskosität bei 5°C von nicht mehr als 90.000 mPa·s.

5. Tensidzusammensetzung gemäß einem der Ansprüche 1 bis 4, wobei das wasserlösliche Salz wenigstens eines ausgewählt aus wasserlöslichen anorganischen Salzen und wasserlöslichen organischen Salzen, die von Tensiden verschieden sind, ist.

6. Tensidzusammensetzung gemäß einem der Ansprüche 1 bis 4, wobei das wasserlösliche Salz wenigstens eines ausgewählt aus Alkalimetallsalzen einer anorganischen Säure und Ammoniumsalzen einer anorganischen Säure ist.

7. Verfahren zum Herstellen einer Reinigungsmittelsammensetzung, das das Einarbeiten eines Tensids und/oder Wasser in die Tensidzusammensetzung gemäß einem der Ansprüche 1 bis 6 umfaßt.

8. Verwendung der Tensidzusammensetzung gemäß einem der Ansprüche 1 bis 6 zur Herstellung einer Reinigungsmittelzusammensetzung.

9. Verwendung gemäß Anspruch 8, wobei ein Tensid und/oder Wasser zusätzlich eingearbeitet wird.

## Revendications

1. Composition tensioactive comprenant les composants (A), (B) et (C) qui suivent :
(A) un polyoxyéthylène éther alkyl sulfate ou alkyl sulfate représenté par la formule (1) suivante :
R¹O(CH₂CH₂O)ₙSO₃M (1)
dans laquelle, R¹ représente un groupement hydrocarbure saturé ou insaturé comportant 8 à 18 atomes de carbone, « n », qui est un nombre moyen de moles ajoutées, est un nombre allant de 0 à 5, et M représente un atome de métal alcalin, une alcanolamine ou de l'ammonium ;
(B) un éther glycérylique ou un éther diglycérylique comprenant un groupement alkyle ou alcényle comportant 4 à 24 atomes de carbone, et
(C) un sel soluble dans l'eau, dans lequel la teneur en Composant (A) n'est pas inférieure à 40 % en poids.

2. Composition tensioactive selon la revendication 1, dans laquelle le Composant (A) et le Composant (B) sont ajoutés à raison d'un rapport en poids (A) / (B) se trouvant dans une plage allant de 99/1 à 75/25.

3. Composition tensioactive selon la revendication 1, dans laquelle le Composant (A) et le Composant (C) sont ajoutés à raison d'un rapport en poids (A) / (C) se trouvant dans une plage allant de 99/1 à 85/15.

4. Composition tensioactive selon l'une quelconque des revendications 1 à 3, possédant une viscosité à 5 °C de pas plus de 90 000 mPa·s.

5. Composition tensioactive selon l'une quelconque des revendications 1 à 4, dans laquelle le sel soluble dans l'eau est au moins l'un choisi parmi des sels minéraux solubles dans l'eau et des sels organiques solubles dans l'eau autres que des tensioactifs.

6. Composition tensioactive selon l'une quelconque des revendications 1 à 4, dans laquelle le sel soluble dans l'eau est au moins l'un choisi parmi des sels de métal alcalin d'un acide minéral et des sels d'ammonium d'un acide minéral.

7. Processus destiné à la préparation d'une composition détergente, qui comprend d'incorporer un tensioactif et/ou de l'eau dans la composition tensioactive telle que revendiquée selon l'une quelconque des revendications 1 à 6.

8. Utilisation de la composition tensioactive telle que revendiquée selon l'une quelconque des revendications 1 à 6 pour la préparation d'une composition détergente.

9. Utilisation selon la revendication 8, dans laquelle un tensioactif et/ou de l'eau est incorporé additionnellement.
